# EUROPEAN PATENT APPLICATION

(11) **EP 1 224 953 A1**
(43) Date of publication of application: **24.07.2002**
(21) Application number: 01830023.6
(22) Date of filing: 18.01.2001
(51) Int. Cl.: A61N 5/01

(54) **Apparatus for the linear acceleration of electrons, particulary for intraoperative radiation therapy**

(71) Applicant: HITESYS S.p.A., 04011 Aprilia (Latina) (IT)
(72) Inventor: Venier, Luigi, 00100 Roma (IT); Santoni, Felice, Dr., 00040 Rocca di Papa (IT); Sagripanti, Alberto, 00042 Anzio(Roma) (IT)

(57) **Abstract**

An apparatus for the linear acceleration of electrons, particularly for intraoperative radiation therapy, including:
- an articulated self-propelled structure (51n), for moving a radiating head (32) that comprises an acceleration structure (39) constituted by plurality of cavities (26);
- a modulator for generating, controlling and transmitting a radio-frequency (34,36), placed in the horizontal arm of the articulated structure (63) that moves the radiating head (32), for the generation, control and sending of the radio-frequency (34,36) to the cavities (26) of the acceleration structure (39);
- an uninterruptible power supply (62) and an automatic control system of the frequency, to power the apparatus independently with respect to the electrical network and to keep the oscillation frequency of the magnetron always equal with that of the accelerator (34,36);
- processing and control devices (30,40) adapted to control the apparatus; the modulator (34,36) is separate from the radiating head (32) and the connection occurs by virtue of wave-guide means (38) adapted to carry the radio-frequency to acceleration structure (32).

## Description

The present invention relates to an apparatus for the linear acceleration of electrons, particularly for intraoperative radiation therapy.

Intraoperative radiation therapy is a therapeutic method used in treating deep neoplasm and consists in delivering a single intense dose of radiation onto a tumoral mass, preventing the dose from affecting the surrounding healthy tissues.

Its flied of utilisation ranges from surgically inoperable tumours, to tumoral residues after partial surgical exeresis, to a tumoral bed after full surgical removal. In this manner, by delivering the dose of radiation directly onto the tumour or onto the macroscopic or microscopic tumoral residue, it is possible to spare the peritumoral healthy tissues that are instead affected by radiation in conventional radiation therapy with external beams.

Currently there is growing interest in the use of this therapeutic method for a wide range of tumours, particularly those affecting the abdomen, the pelvis, and the chest. The association of this therapy with surgery and with conventional radiation therapy allows to considerably improving local control of advanced-phase neoplasm.

Large electron accelerators, which allow to treat a patient both with an electron beam and with X rays, are utilized with hunges operative and logistical difficulties in the execution of intraoperative radiation therapy. Use of electron-beam therapy offers high versatility in treating tumoral residues after surgical removal as well as tumoral masses deemed inoperable.

An extraordinary contribution, for the utilisation more appropriate, in accordance with the demand and the necessity radiotherapeutic current, particularly for direct use of the linear acceleration inside operating room, as been given to the specific field radiotherapeutic from the "apparatus for the linear acceleration of electrons, particularly for intraoperative radiation therapy", which patent N° 01281184, awarded in Italy in date 17.02.1998, N° 5635721, awarded in U.S.A. in date 3.06.1997, and to the application European Patent Office N° 95114537.4, date of receipt 15.09.1995.

The patent recalled and appointed has intend eliminated very much disadvantages and/or risks for the use of the linear acceleration electrons, in the specific field of the intraoperative radiation therapy, which:
-- the need transfer the patient to a location other tan the operating room causes problems linked to the risks of transferring the patient under anaesthesia and to the time that elapses between surgical exeresis and subsequent radiation therapy;
-- the need to strictly schedule each operation according to the availability of access to the radiation-therapy site, and this increases the working time requirements ad reduces the number of patient who can utilise this radiation therapy;
-- the high manufacturing cost and the remarkable encumbrance of the apparatus; the impediment to placed the radiating head in space and to be moved in a flexible manner, so that the electron beams treats the entire tumoral mass involved, despite the irregular shape that said mass may have;
-- the elimination of the intense irradiation produced, which cannot be confined with simple movable panels, and therefore in the consequent need for heavily shielded work sites.

For the elimination of the disadvantages and/or risks, the patent ahead recalled shows and shape the realisation of the apparatus for the linear acceleration of electrons, particularly for intraoperative radiation therapy, that can by used directly in the operating room without special radiation-protective measures; that allows the flexible and precise movement in space of the electron beam to treat tumoral masses having variable and different shapes; that allows to provide the electron acceleration section separately from the radio-frequency generation ad control section; that allows to achieve a very low X-ray level that can by easily shielded; that allows to spatially vary the dose of radiation that is incident to a given tumoral area; having a modest size and weight; that avoids the need for the external focusing an centring devices for the emitted electron beam.

All the innovations of the patent, ahead recalled and appointed, and that in the specific flied of the itraoperative radiation therapy define the current state of the technique, find in the present invention all indication of the improvement of the functional features and/or of the functional requirements in reply to present needs of the itraoperative radiation therapy.

Obvious, the apparatus that way conceived and patented is capable of improvement of the functional requirements, as he writes in the application; improvement that present invention show in the description.

A principal aim of the present invention is therefore to provide an apparatus for the linear acceleration of electrons, particularly for intraoperative radiation therapy, which can be used directly in the operating room without special radiation-protective measures, that, on the grounds of the techniques fits in the patent ahead called, introduce other technical elements more advanced for the realisation of the apparatus more satisfactory for the running, furniture, autonomy and control of the process radio-therapeutic, whatever proposals may be.

Within the scope of this aim, an object of the present invention is to provide an apparatus for the linear acceleration of electrons that allows too of to enliven through radio control the whole articulated structure, in order to manage jointly the radiating head, but also shift's comfort outside and in operative room, for allows better the flexible and precise movement in space of the electron beam to tat tumoral masses having variable and different shapes.

Another object of the present invention is to provide an apparatus for the linear acceleration of electrons feed from uninterruptible power supply, that give called apparatus independent, during therapeutic process, from the power supply network for to avoid interference and/or troubles of the network to the functions of the same apparatus.

A further object of the present invention is to provide an apparatus for the linear acceleration of electrons having the frequency modulator in the arm horizontal of the robot. Such placing allow a better flexibility and a certain balance of the apparatus.

Another object of the present invention is to provide an apparatus for the linear acceleration of electrons having a automatic frequency control, that objet is to keep the oscillation frequency of the magnetron always coincidental with that of resonance of the linear accelerator of electrons structure.

This aim, these objects, and others which will become apparent hereinafter are achieved by an apparatus for the linear acceleration of electrons, particularly for intraoperative radiation therapy, characterised in that it comprises:
-- an articulated structure for moving irradiation means that comprise an acceleration structure constituted by a plurality of cavities;
-- modulation means for generating, controlling and transmitting a radio-frequency to said cavities of said acceleration structure; and
-- processing and control means adapted to control said apparatus, said modulation means being separate from said irradiation means, the connection occurring by virtue of waveguide means adapted to carry the radio-frequency to said acceleration structure.

Further characteristics and advantages of the invention will become apparent from a preferred but not exclusive embodiment of the apparatus to the invention, illustrated oily by way of non-limitative example in the accompanying drawing, wherein:
figure 1 is a view of a known type of apparatus for the linear acceleration of electrons;
figure 2 is a view of the configuration of the acceleration cavities for a known type of acceleration apparatus;
figure 3 is a block diagram of a known type of an apparatus for the linear acceleration;
figure 4 is a block diagram of automatic frequency control system according to the invention;
figure 5 is a lateral elevation view of a robot and of radiating head connected thereto, said robot and said head being a part of acceleration apparatus of a known type;
figure 6 is a lateral elevation view of a robot and of radiating head connected thereto, said robot and said head being a part of the acceleration apparatus, and a uninterruptible power supply according to the invention;
figure 7 is a flowchart of the steps for the characterisation of area of the body of a patient to be treated with the apparatus of a known type;
figure 8 is a flowchart of the operating steps for scanning irradiation by using the apparatus of a known type.

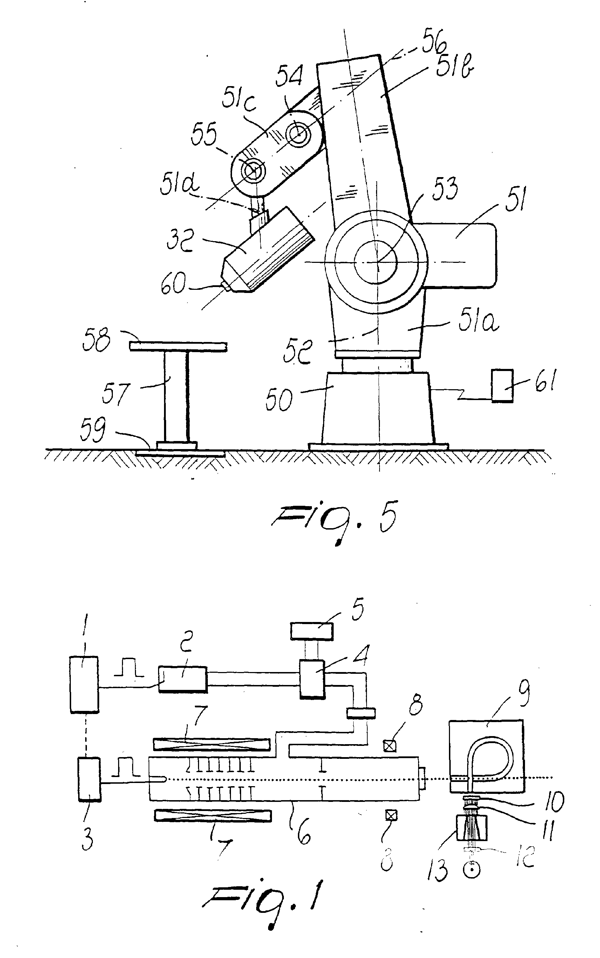

With reference to figure 1, the known acceleration apparatus comprises a radio-frequency modulator 1, a magnetron 2, cathode modulation means 3, circulation means 4, a load cooling water 5, a acceleration structure 6, a focusing magnet 7, a centring magnet 8, a deflector magnet 9, a beam diffuser 10, a beam equaliser 11, a beam applicator 12, and a beam collimator 13.

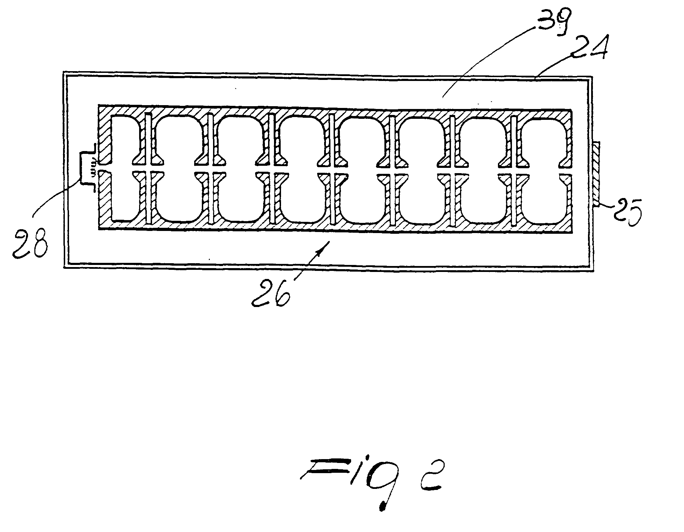

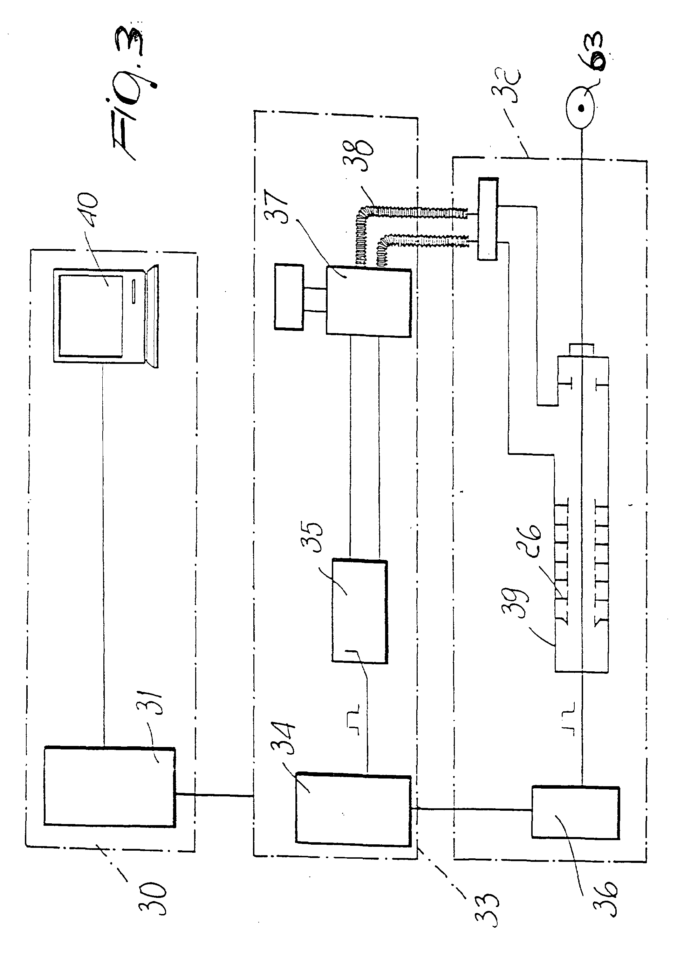

With references instead to figures 2 and 3, illustrates in detail the known and patented acceleration structure 26, which is constituted by a plurality of acceleration cavities 26 ( also known as resonant cavities), arranged in series one next to the other, by a cathode 28 connected to the first acceleration cavity and by control and processing means 30. The acceleration cavities 26 are enclosed by an external vacuum-tight jacket 24.

The acceleration cavities 26 of the acceleration structure of a known type 39 are designed so as to produce radio-frequency self-focusing along the X-axis of said cavities.

Self-focusing has bee obtained by using different length values for the first, second, third, fourth and fifth acceleration cavities, so that the lengths increase from the first cavity to fifth one and are constant for the subsequent cavities. Particularly, it has been found that the optimum lengths for the first, second, third, fourth an fifth cavities are 25 mm, 40 mm, 45 mm, 48 mm and 50 mm, respectively. The energy of the electrons and their capture have also been treated for the first cavity, in which the electrons are not yet relativistic ad the values of b and r (Lorenz parameters) vary appreciably.

A cathode 28 is placed in front of the first acceleration cavity and is supplied by the cathode modulation means 36; a thin titanium lamina is arranged outside the last acceleration cavity for vacuum tightness.

The control and processing means 30 comprise: power supply means 31; processing means 40, which advantageously comprise computer; the cooling system of the apparatus; the means for distributing motive power; the safety devices (not shown).

The control and processing means 30 are connected to modulation means 33 that comprise radio-frequency modulation means 34 connected to magnetron 35 and to cathode modulation means 36. The magnetron 35 is protected from accidental load reflections by a ferrite isolating system 37. Waveguide means 38, conveniently constituted by a flexible waveguide, connect the radio-frequency modulation means 33 to irradiation means, constituted by a radiation head 32 that comprises an acceleration structure 39.

In figure 3, the reference numeral 63 designates a particular area of the body of the patient that must be treated by irradiation by means of the apparatus of a known type and according to the invention.

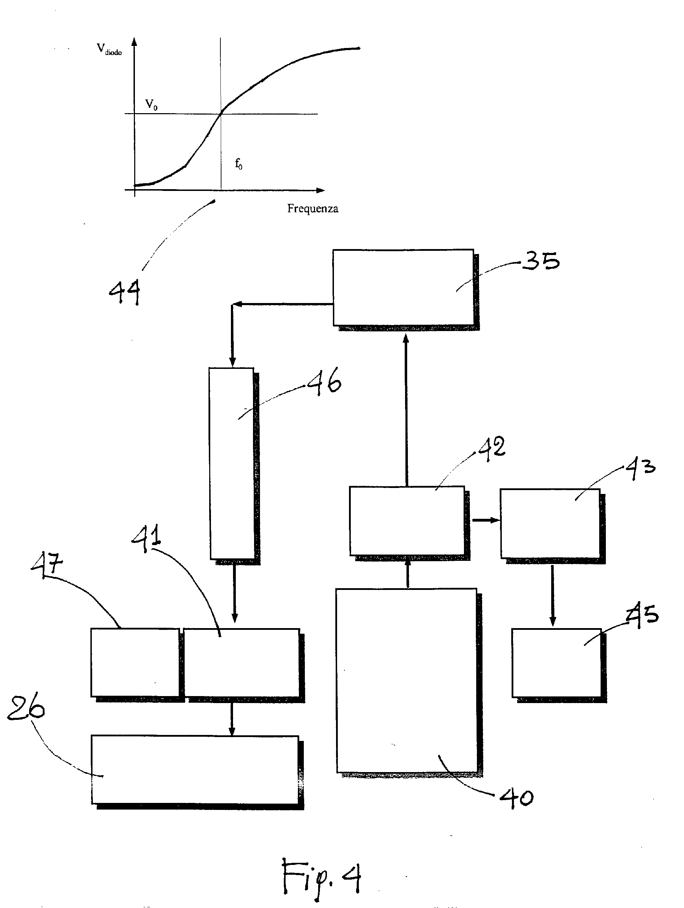

With reference instead to figure 4, the acceleration apparatus according to the invention comprises also the radio-frequency self- control, of which object to keep the frequency of oscillation of the magnetron 35 coinciding with that of the resonance of the acceleration structure 26.
In according of the known type of the apparatus, as a matter of fact, the magnetron 35 and the acceleration structure 26 undergo to frequency individual deviations of the work point ( within limits), what for example the temperature, what influences the resonance frequency.

The present invention eliminate such disadvantage by means of a self-control circuit of the frequency 40 that provides to keep in step the oscillator and load.

A capacity coupling 41, placed in the knot of the wave guide 38, draws a share of the radio-frequency. In fact, the feature of the knot point is that to present a behaviour of the voltage highly depending from the type of coupling oscillator-load; such dependence is showed in the graph 44. In the abscissas axis are carried the emission frequencies of the magnetron 35; in the middle of the axis is gave the resonance frequency of the accelerator f₀. The axis of ordinate represents the voltage took from the diode joined with the capacity coupling 41: peak value of the voltage present in the wave-guide 38, in the knot point where the coupling is inserted.

To vary of the frequency of the magnetron, the voltage took from the diode to vary around a central value V₀; confronting afterwards the voltage of the diode with an voltage equal to V₀, it is possible to dispose of a signal right for to drive the motor that the syntony axis, 42; the rotation towards is that what stretch to carry V toward V₀ and so therefore the frequency f toward f₀.

Afterwards the peculiar characteristic of the frequency self-control circuit analysed is that to make use only one signal drew from the wave guide, unlike of the known system and of all the other sisters that use 2 signals.

Figure 5 is a lateral elevation wives of the apparatus of a known type in which the reference numeral 50 designates a supporting structure fixed to the floor or resisting thereon.

An articulated structure 51 is located on the supporting structure 50 and is meant to support and move the radiating head 32; a diaphragm 60 is applied to said radiating head in the position where the electronic beam exits.

In detail, the articulated structure 51, commonly termed tube support, is constituted by a vertically arranged robot comprising four articulated and mutually interconnected segments designated by the reference numerals 51a, 51b, 51c and 51d, which allow to arrange the radiating head 32 in any spatial position.

The articulated segments 51a, 51b, 51c and 51d are mutually pivoted so as to give the radiating head 32 six degrees of freedom in space.

In particular, the articulated segment 51a is routable about the rotation axis 52 of the supporting structure 50 both clockwise and counterclockwise; the articulated segment 51 b is routable in both directions about the hinge axis 53; the segment 51c is routable in both directions about the hinge axis 54 as well as about the axis 56; and the segment 51d is routable in both directions about the hinge axis 55.

The reference numeral 57 designates a supporting post for operating table 58. Said post rests on a footing 59 that is moderately shielded, with respect to the floor, only if access to the rooms beneath the operating room is not prohibited during use of the apparatus of a known type. Said shielding is not necessary otherwise.

Control means, advantageously comprising a movable button panel 61, control the apparatus of a the known tie.

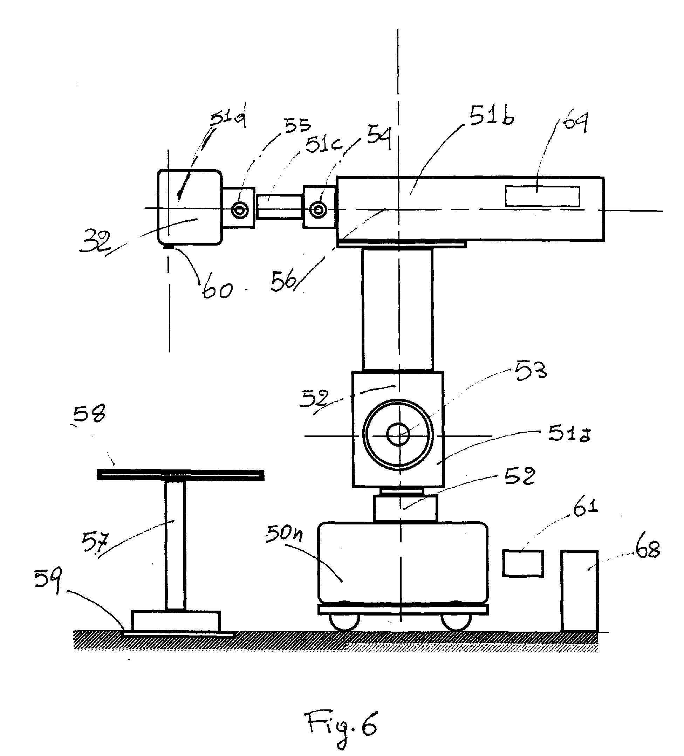

Figure 6 is a lateral elevation view of the apparatus according to the invention, in which the reference numeral 50n (new) designates a supporting structure with carriage self-propelled and radio controlled as all other components of the supporting structure of the radiating head 32, that keeps six degrees of freedom in space, as in figure 5.

Considering that the base principles of operation delineates of the known type of the apparatus, the reference numerals previously describes they are just the same thing, while are carried the reference numerals regarding the functions or the inserted parts of the present invention.

As regards, the figure 6 show the better placing of the modulator (34,36), housed in a metal container entirely closed 64 placed in the horizontal arm of the articulated structure, for the generation of the radio-frequency to send to the cavities 26 of the articulated structure of the robot 39.

Furthermore, with the reference numeral 62 is showed the uninterruptible power supply for the self-power supply of the apparatus, of which fundamental purpose is to give the operation of the apparatus free from the electrical perturbations of the network, during the its use radio-therapeutic.

And some more, although not showed in figure 6, for the improvement that to object the present invention, has been a thermostatic circuit with a exchanging heat air-fluid instead of water-fluid. Obvious, the fluid used for the cooling is alone that of the shunt circuit of the apparatus and not there are necessities of the external intake.

After all that, with reference to figure 6, has been the numbers of the part-functions inserted and/or altered from the present invention, preserving the numeration of the part-functions not altered, as in figure 5.

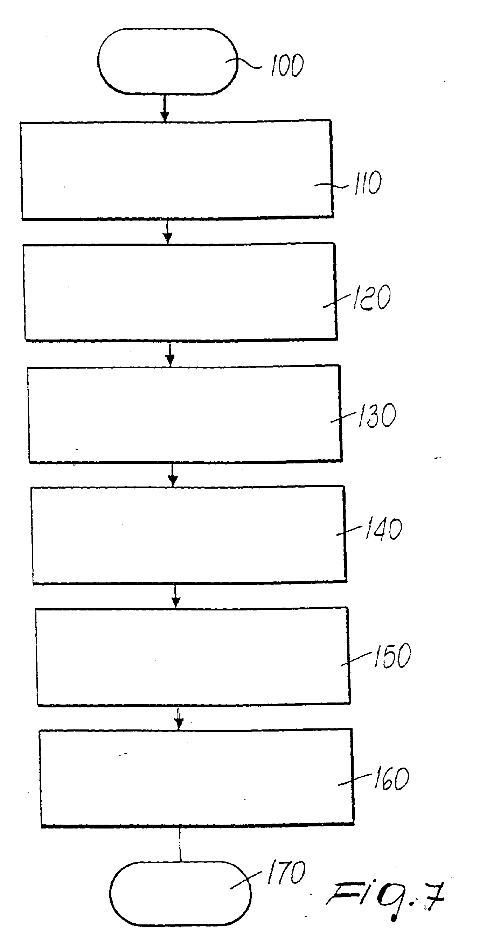

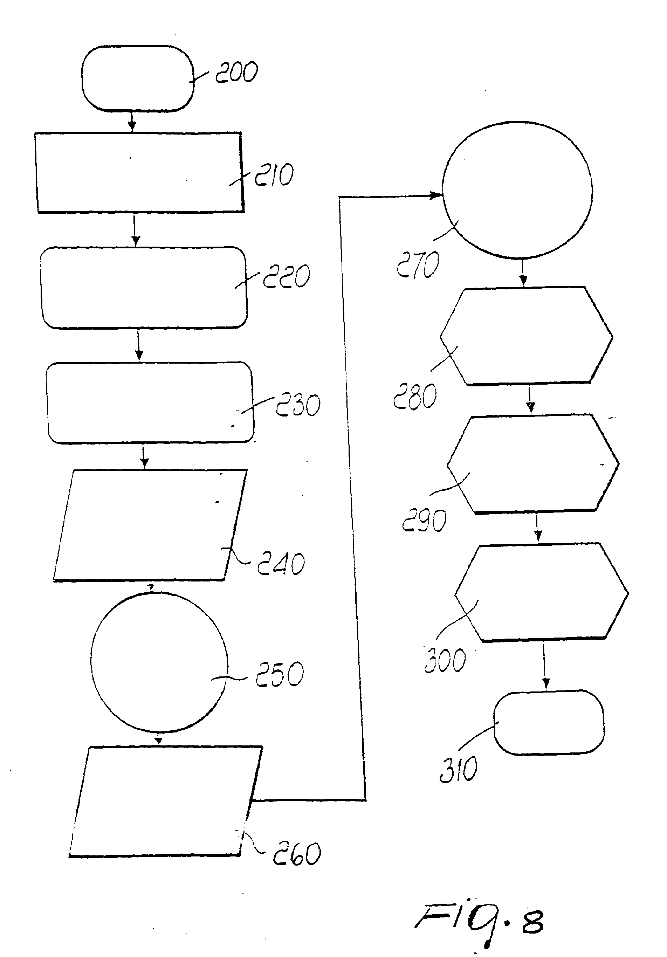

Figures 7 and 8 are flowcharts of the operating steps of the apparatus according to the invention, which are handled by processing means 40.

With reference to the above figures, the operation of the apparatus for the linear acceleration of electrons according to the invention is as follows. The robot constituted by the articulated segments 51a, 51b,51c and 51d allows to orientates the radiating head 32 so as to direct the electron beam exactly onto the region where the therapy is to be performed. The possibility of orientating the radiating head 32 allows to avoid expanding the beam and therefore allows precise treatment of the region to be irradiated, bringing the radiating head 32 very close to the region to be irradiated. This, in comparison with the minimum possible distance that can be obtained with known linear accelerators, which varies from 80 to 100 cm, allows the apparatus according to the invention to have a much higher efficiency of the aid patent, since there is no beam scattering and a much lower power level is thus required.

Furthermore, the carriage supporting structure self-propelled and radio controlled 50n, according to the invention, consent the approach of the apparatus to the supporting post for operating table 58. Shifts what are radio-controlled and monitored respect to tumoral area of the patient that must be took by irradiation to the apparatus of the known type and according to the invention 63.

Continuous measurement of the position of the various articulated segments that constitute the robot occurs by means of a sensor system (not )shown) with which the robot is equipped. The processing means 40 allow to predefine the desired movements of the articulated segments 51a-51d that constitute the robot, and therefore of radiating head 32, so that said radiating head closely follows the contour of the region to be irradiated; it is furthermore possible to set the desired doses of radiation for each point of the region to be treated.

The modulation means generate and control the radio-frequency and feed to the cathode 28. The generated and self-controlled radio-frequency 40 is sent to the acceleration cavities by means of the flexible wave-guide 38.

The electrons that move along the axis of the acceleration cavities 26 are gradually accelerated by the radio-frequency flied inside each cavity 26 until they reach the desired final energy. The electrons exit from the acceleration structure 32 through the thin titanium lamina 25, the thickness whereof allows the electrons to pass there through without losing an appreciable part of the energy they possess.

The radio-frequency electric field used to accelerate the electrons is produced by the magnetron 35, which feeds the acceleration structure 32 by means of the wave-guide 38.

The modulator of the cathode 36 feeds the cathode and synchronises its operation so that the train of radio-frequency pulses that feeds the acceleration structure 32 is matched by emission of electrons on the part of the cathode.

The beam of electrons is focused an accelerated simultaneously in the first cavities with a set combination of the cathode injection energy and of the length of the first, second, third, fourth an fifth cavities, and can pass through the centre of the subsequent cavities of the acceleration structure after the peak of the radio-frequency, so as to undergo additional focusing.

The beam of electrons is pulsed, and each pulse lasts 4 microseconds. The frequency of the pulses can be fixed or variable.

Use of self-focusing of the electron beam allows to eliminate auxiliary devices used in known accelerators, and in this manner the electrons beam does not encounter metallic masses along its path and therefore does not produce radiation, allowing to use the apparatus according to the invention directly in the operating room without particular protective measures.

Use of the processing means 40 allow to use the apparatus according to the invention for mechanical scanning irradiation comprise four different operating states of the acceleration apparatus.

These four states are:
-- instruction step;
-- learning step;
-- verification step;
-- therapy step.
Figure 7 is a block diagram of the sequence of steps for defining an area to be irradiated, which is called "source plane", performed by the processing means 40; in said figure, after the initial step 100, there is a step 110 for selecting the plane on which the area to be irradiated lies; the step 110 is followed by the step 120 for selecting the inclination angle of the radiating head 32 with respect to the defined source plane; this is then followed by the step 130 for entering the vertices of the figure to be irradiated, the step 140 for tracing the perimeter of the figure for confirmation, the step 160 for calculating the data for irradiation, the irradiation step 150, and finally the end step 170.

With reference now to figure 8, when the patient is ready for electron therapy ( step 200), the apparatus according to the invention is placed (step 210), by opening the articulated structure 51, so that its beam direction indicator is trained on the centre of the area to be treated. This movement is controlled by means the movable bottom panel 61.

At this point, the apparatus is placed in the learning state: the operator starts to move the beam over a path that coincides with the edge of the region to be treated; this path is developed over more or less spaced points, depending on the complexity of the profile, and the processing means 40 interpolate an connect the various points with straight segments or circular arcs.

Once learning has ended, the apparatus is placed in the verification step, during which the operator enters ( steps 240 and 260), the required dose and the corresponding energy for each field and the articulated structure 51 constantly moves ( steps 250 and 270) along the corresponding paths with its light beam.

Once the verification step has ended, the steps for the irradiation of the various fields (steps 280, 290 and 300) occur. These irradiation steps can be followed on the monitor of the processing means 40, which displays data related in real time to the path in progress, the percentage of treatment performed, the dose given, and the remaining dose.

This is followed by the end step 310.

In the case of conventional irradiation, the operator, after moving the radiating head 32 close to the patient, and after setting the desired dose by virtue of the processing means 40, moves the radiating head 32 towards the collimator cone that is used to protect the parts of the patient's body that are non to be irradiated from the electrons beam, and begins the irradiation.
The separation between the radiating head 32 and the modulation means 33, and their connection by means of the flexible wave-guide 38, allow to provide an articulated mechanical arm 51 capable of positioning and moving in space the acceleration structure 39 and the radiating head 32 with extreme precision and in very flexible manner. Control by means of an appropriately programmed computer 40 allows to set up the movement of the articulated structure 51 so that the beam of electrons treats the entire tumoral mass, no matter how irregular it might be.

The beam self-focusing characteristic, combined with the shape of the acceleration structure 32, make it unnecessary to have auxiliary external devices for regulating the beam (magnets, etceteras), thus allowing a very low X-ray level. This reduction in radiation allow using the apparatus in operating rooms without particular shielding.

The use vacuum-tight welding to assemble the various acceleration cavities 26 that constitute the acceleration structure 32 allows eliminating the external vacuum-tight jacket, thus reducing weight and size.

The device thus conceived is susceptible of numerous modifications and variations, all of which are within the scope of the inventive concept. Thus, for example, since in the apparatus according to the invention the cross-section of the beam remains very small, the current of the beam must in turn be reduced considerably, on penalty of risking the delivery of an excessive local dose and therefore necrotizing the irradiated tissue. The need to reduce the intensity of the current can allow eliminating the cathode 28 from the acceleration structure 39 by virtue of known phenomenon of cold extraction of electrons from a metallic material. This phenomenon consist of the fact that an intense electric field applied to a metallic material is able to extract a certain number of electrons from the outermost atomic orbits. The number of electrons that can be extracted, however, is not sufficient for the beam currents required by known accelerators: with certain exception, as the patent ahead mentioned.

Finally, all the details may be replaced with other technically equivalent elements. In practice, the materials employed, so long as they are compatible with the specific use, as well as the dimensions, may by any according to the requirements and state of the art.
Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An apparatus for the linear acceleration of electrons, particularly for intraoperative radiation therapy, **characterized in that** it comprises:
-- an articulated structure self-propelling for moving irradiation means that comprise an acceleration structure constituted by a plurality of cavities;
-- modulation means for generating, self-controlling and transmitting a radio-frequency to said cavities of said acceleration structure;
-- processing and control means adapted to control said apparatus, said modulation means being separate from said irradiation means, the connection occurring by virtue of wave-guide means adapted to carry the radio-frequency to said acceleration structure.

2. An apparatus according to claim 1, **characterized in that** said wave-guide means comprise a flexible wave-guide adapted to connect said modulation means to said irradiation means.

3. An apparatus according to claim 1, **characterized in that** said articulated structure comprises a robot for the movement of said irradiation means, said robot being arranged on a mobile supporting structure self-propelling above the floor of the operating room.

4. An apparatus according to claim 3, **characterized in that** said robot comprises four articulated segments that are movable with respect to each other and allow said irradiation means to move with six degrees of freedom.

5. An apparatus according to claim 1, **characterized in that** it comprises processing means adapted to manage said apparatus, said processing means being connected to said modulation means.

6. An apparatus according to claim 1, **characterized in that** said plurality of cavities of said acceleration structure are interconnected by means of vacuum-tight braze welds, the cavities from the first to the fifth one having increasing lengths, the subsequent cavities being identical in length, said plurality of cavities producing a self-focusing of the electron beam.

7. An apparatus according to claim 6, **characterised in that** said acceleration structure comprises a cathode located in front of said first cavity and titanium lamina located outside the last cavity.

8. An apparatus according to claim 1, **characterized in that** said modulation means comprise a magnetron, a radio-frequency modulator, and a cathode adapted to enable said cathode.

9. An apparatus according to claim 1 and 2, **characterized in that** said modulation means utilise alone one signal drew to the wave-guide; unlike of the all the other systems that about utilise two.

10. An apparatus according to claim 3, **characterized in that** said articulated structure is self-propelled and, afterwards, adaptable to the placing of the tables of the operating room.

11. An apparatus according to claim 6, **characterized in that** said acceleration structure comprises a titanium lamina located outside the last cavity.

12. An apparatus according to claim 1, charecterized in that said modulation means comprise a modulator of radio-frequency placed in the horizontal arm of the articulated structure.

13. An apparatus according to claim 9, **characterized in that** said modulation means comprise a automatic control of the frequency deed to keep the oscillation frequency of the magnetron coinciding with that the resonance of the acceleration structure.

14. An apparatus according to claim 1, **characterized in that** said power supply is independent, during the use, from the electrical network and the said hydraulics system is independent for the thermostatic circuit, because need not external intakes for the cooling.

15. A process for performing intraoperative radiation therapy by using the apparatus for the acceleration of electrons defined in claims 1 to 14, **characterized by** the steps that consist of an instruction step, a learning step, a verification step, and therapy step.

16. A process according to claim 15, charecterized in that said instruction step consists in making said apparatus follow manually the perimeters of the areas to be treated; in that said learning step consists in storing said perimeters on the part of said apparatus; in that said verification step consists in verifying said paths and in simultaneously entering the parameters that indicate, for each path, doses and energy of the electron beam used for the treatment; and in that said therapy step consists in performing the irradiation of said defined areas according to said set doses an energy.
